# EUROPEAN PATENT SPECIFICATION

(11) **EP 2 611 362 B1**
(45) Date of publication and mention of the grant of the patent: **03.08.2016**
(21) Application number: 11767648.6
(22) Date of filing: 01.09.2011
(51) Int. Cl.: A61B 5/151, A61B 5/1486

(54) **DEVICE FOR CONTINUOUS MEASURING OF AN ANALYTE**
VORRICHTUNG ZUR KONTINUIERLICHEN MESSUNG EINES ANALYTEN
DISPOSITIF DESTINÉ À MESURER UN ANALYTE EN CONTINU

(30) Priority: 01.09.2010 ES 201031309
(43) Date of publication of application: 10.07.2013
(73) Proprietor: Garcia Saban, Francisco Javier, 15530 Cerdido (A Coruña) (ES); Marsinyach Montfort, Jordi, 08271 Artes (Barcelona) (ES)
(72) Inventor: Garcia Saban, Francisco Javier, 15530 Cerdido (A Coruña) (ES); Marsinyach Montfort, Jordi, 08271 Artes (Barcelona) (ES)
(74) Representative: Carpintero Lopez, Francisco
(86) International application number: PCT/EP2011/065076
(87) International publication number: WO 2012/028675

(56) References cited:
- WO-A1-03/088851
- WO-A1-2010/011805
- WO-A2-2007/041244
- WO-A2-2007/041287
- US-B1- 7 225 008

## Description

### Object of the Invention

As indicated by its title, the object of the invention relates to a device for continuous measuring of an analyte which allows continuous monitoring of the blood or interstitial glucose level in a minimally invasive, minimally detrimental and painless manner.

Said device, which allows measuring in real time, likewise comprises systems for managing, processing and telematically linking data to external reading means with two-directional communications such that it allows sending commands to the processor of the fixed part to reprogram and configure the operation of the puncturing units for sampling the analyte, according to the evolution of the patient and according to the guidelines established by the doctor.

### Background of the Invention

As is known, Diabetes Mellitus (DM) is a metabolic disease which is characterized by a persistent increase of glycemia (blood glucose) above 125 mg/dl. This increase of the blood sugar (hyperglycemia) is the cause of the acute and chronic complications of the disease.

Diabetes mellitus is a common disease and it increases more each day. The problem is caused by different factors, the most significant being: the inheritance and the action of acquired factors, unhealthy habits which lead to obesity the cause of which is usually physical inactivity (lacks of physical exercise), and a fat and sugar rich diet. Among other elements which are responsible for the metabolic syndrome which tends to lead in many years to the onset of diabetes include at the same time other factors such as arterial hypertension, the increase of fats in blood, smoking habit and stresses.

In the last decades an extraordinary increase of the diabetes mellitus cases has been produced worldwide. It is estimated that the diabetic population is duplicating every 15 years, causing a negative impact on the global health quality; with severe personal, family and social repercussions. This disease is a serious and very costly public health problem which does not discriminate ages or socio-economic levels. It is necessary to put-up a containment barrier against that expected avalanche of diabetics and their complications. This task must be carried out by means of a suitable primary prevention structure and an exhaustive control of the disease such as secondary prevention.

With the treatment and successive controls by means of formation, self control and those made by the doctor, fasting blood sugar ideally lower than 120 mg/dl and at one hour after eating lower than 160 mg/dl must be achieved.

Glycemic control is fundamental for controlling diabetes, therefore the self-monitoring of glucose (AGS) forms a primordial and effective component within the factors surrounding disease control providing the patients with an individual evaluation system for evaluating the response to his/her individualized therapy together with the other guidelines of his/her environment.

The AGS scanning and its frequency must be established after an individualized study of the patient and his/her environment, providing confidence, necessity and clear benefit, being fundamental in patients with type I diabetes and with guidelines adapted to type II patients, closing the circle of the variables of the disease.

Current standard diabetes control systems use self-analysis of blood glucose and urine glucose (currently displaced by that of blood) by means of reactive strips having the incorporated glucose reagent.

The self-analysis of blood sugar by means of reactive strips requires first positioning the reactive strip in an equipment called glucometer (which measures and gives the result of the amount of glucose) and positioning the self-puncturing device by means of a blood lancet or needle in the fleshy part of the finger to obtain a drop of blood and to deposit it in the inlet of the strip.

There are 2 types of glucometers, according to the technique used:
Reflectometers: they measure the light reflected from the reagent after it has gone through a chemical reaction (enzymatic oxidation of the glucose). In the reactions a chromatic product is produced. The intensity of the color is proportional to the amount of glucose present.

Electrochemical biosensors: they measure the electric current generated in the enzymatic reaction of glucose oxidation with the reagent.

These self-analysis by means of the reactive strips located in glucometers have a series of advantages including:
- They facilitate the modifications of the treatment by the doctor.
- They facilitate self-adjusting the treatment depending on the daily circumstances and thereby the self-control of the patient.
- They prevent and detect hypoglycemia.
- They detect and treat severe hyperglycemia.
- They improve appeal and therefore facilitate compliance.
- They provide autonomy to the patient and his/her family.

However, there are also a series of drawbacks meaning that this technique is not the best for the whole world. Among the more common drawbacks that can be found are the followings:
- It is a bloody technique: produces pain in pricked areas, more or less significant injuries which must be cured and with risk of infection.
- It requires empowerment: visual acuity, dexterity, training, etc.
- Possibility of errors in the readings.
- It needs cleaning the glucometer.
- The glucometer must be calibrated.
- It does not allow making semi continuous measurement at every certain time since blood lancets have to be used and it causes injuries which require time to heal, it wears out the healthy areas of puncture.
- There is the possibility of manipulating the results.
- The cost of the control system.

To solve some of these problems in the recent years, different systems have been developed for determining over time with more or less frequency, the values of glucose in the so-called interstitial fluid, i.e., at subcutaneous level.

These systems however in no case substitute the common measurement of capillary blood sugar due to the fact that the determination of the interstitial glucose entails a delay with the figures of the blood glucose level, using mathematical algorithms being necessary to determine the figure of blood glucose which was there some time ago. Nevertheless, the published results of the studies performed prove the observation of more hyperglycemia peaks as well as hidden hypoglycemia peaks when measurements at interstitial level are used than when the common method for measuring capillary blood sugar is used, leading one to think that the determination of blood glucose is a temporal method of the figures of blood sugar, but incomplete from a scientific point of view. It must be mentioned that from a physiological point of view the control in the blood sugar regulation is established from glucose located in interstitial space and from its specific transport (GLUT2) into the β-cells of the pancreas.

On the other hand, there are the so-called "monitoring system" which attempt to monitor the evolution of the glucose levels for several days to detect hypoglycemia, specially at night, the hyperglycemia peaks and oscillations following a similar hourly pattern and thus adjusts the insulin treatment, involving an advance use of personalized algorithms to automatically modify the infusion dose from the insulin pumps.

Currently, there are many glucose monitoring system which could be grouped according to the characteristics thereof. These characteristics include:
a) Harmfulness.
   Not harmful: they measure the glucose without traversing the skin.
   Semi harmful or semi invasive: they produce a minimum breakage of the external skin layers; and
   Surgical: They require locating the sensor surgically.
b) Analysis system.
   Enzymatic reaction: glucose in contact with a substance is oxidized, an electric charge which is proportional to the amount of glucose being generated.
   Microdialysis: A "U"-shaped micro fiber in inserted under the skin, which micro fiber is connected to a circuit bringing in a liquid collecting the glucose, measuring the amount in an external apparatus (monitor) by an enzymatic system.
   Reverse iontophoresis: an electric charge brings the glucose to the surface of the skin wherein it is analyzed by an enzymatic method; and Infrared: the "C"-shaped detector is surgically implanted around a vessel of 4-5 mm in diameter.
c) Frequency and duration of the registration:
   The determination frequency varies generally between every 3 and every 20 minutes and the duration of the registration goes from 12 hours to more than 3 days in the unharmful or minimally harmful systems to unlimited in the intravascular surgical insertion systems.
d) Calibration:
   All the monitoring system need to incorporate to its register at least one blood sugar value, generally capillary blood sugar value to be used as a reference and to be calibrated. The number of blood sugars needed varies from 1 to 4 in 24 hours.

However, the current systems for monitoring the glucose levels over time are based on the sampling at interstitial level, with the aforementioned problems, to which the short average life of the sensor which tends to be between 3 and 7 days, the exaggeration at the times of being placed or the fact that they tend to need the change of puncture site to prevent cutaneous or other pathology reactions needs to be added.

Devices and systems based on some of the preceding techniques which attempt to solve some of the mentioned drawbacks with the aid, for example, of micorsystems such as micro-needles for puncturing are known in the state of the art.

In this sense, the Spanish patent application 200402373 based on a microsystem for the controlled extraction and injection of fluid can be mentioned for example. This system relates to the extraction of interstitial fluid, i.e., the fluid found in the extracellular space between one cell and another at a depth which is usually less than 0.5 mm, not penetrating the surface vessels which are usually found at a distance between 1 and 4 mm. Thus, since there is a negative pressure under the epidermis with respect to the outer part of the skin, a sample extraction mechanism is necessary to compensate said pressure difference. On the other hand, since the amount of sample extracted by this system is very small, said document describes the use of several micro-needles each time.

Another example described in the state of the art is that of the European patent EP1144078, in which it describes a cartridge with a plurality of micro-needles all of which penetrate the patient at the same time collaborating the extraction of a single sample, therefore said cartridge being of a single use. In this case said cartridge is intended to extract a sample at blood vessel level, i.e., not needing any extraction system since the breakage of said blood vessel and the extravasation of blood increases the inner pressure with respect to the outer pressure, causing the ascent of the sample through the needle by capillarity. Other examples of the state of the art can be seen on WO2007041287, US7225008, WO2010011805 and WO2007041244.

### Description of the Invention

The device for sequential measurement of the present invention solves the aforementioned problems, forming a device capable of performing, in a temporal manner, a continuous temporal monitoring of blood glucose or interstitial glucose by means of a minimally invasive, minimally detrimental and painless system, allowing the measurement in real time and including telematic link systems for linking data to an external reading means and transmitting the dose of insulin to be administered to dispensation devices.

To that end, said device comprises a fixed element which is placed on the user's skin by means of a suitable adhesive means, mechanical means, etc, and comprising an intelligent core or processor and a disposable exchangeable element which is coupled to the fixed element and which incorporates the system performing the samplings.

Said system for sampling consists of an automated mechanical multi-puncturing system comprising micro-needles, the means for driving said micro-needles, the samples of reagent for mixing with the analyte, the electrodes for the reading and the electric contacts necessary for the operation of the driving means and the suction system, and wherein each of the puncturing units is susceptible to be programmed for its activation in a determined time instant and at the depth necessary according to the type of sample to be obtained, i.e., if the measurement is made in blood or in interstitial fluid. Likewise, the system will also decide, depending on the type of measurement to be made or on other pre-established criterion, the start and stop of the sample suction system operation.

Thus, once said measurements are made, these are sent at the first instance to the processor to obtain the data of the blood glucose level or interstitial fluid, performing the transfer of these data in a second instance to the database and to the communications system inside the device of the invention which in turn will communicate and send the data to the external reading and management device.

More specifically, the fixed element of the device of the invention comprises the electronic part for controlling the disposable element which in turn comprises:
a. a processing and control unit of the mechanisms for activating and deactivating the driving means and the suction system.
b. a data processing unit which allows capturing analog readings coming from a interstitial or blood glucose measurement system.
c. an intelligent core for gathering, translating, interpreting and transmitting the
   data (RDTIT) to a external receiver device.
d. an integrated circuit for wireless communication.
e. a multiplexer system for operating the means for driving the micro-needles.
f. a multiplexer system for selecting the different sensors and routing the data towards the processor; and
g. at least one battery for supplying power to the system.

### Description of the Drawings

To complement the description and for the purpose of aiding to better understand the features of the invention according to a preferred embodiment thereof, a set of drawings is attached as an integral part of said description in which the following has been depicted with an illustrative and non-limiting character:
Figure 1 shows a schematic plan view of the device for continuous measurement of the invention.
Figure 2 shows a cross-section elevational view of one of the puncturing units of the device of the invention.

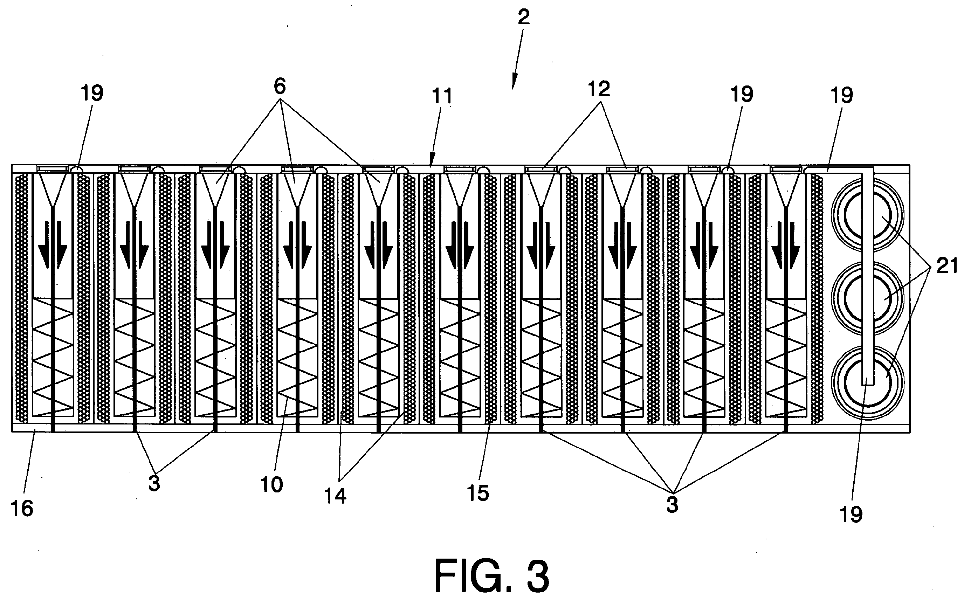

Figure 3 shows a cross-section elevational view of the device of the invention, in which a row of puncturing units is seen.
Figure 4 shows a schematic upper plan view of the lower cover of the device of the invention.
Figure 5 shows a cross-section elevational view of the intermediate layer of the device of the invention.
Figure 6 shows a schematic upper plan view of the upper cover of the device of the invention.
Figure 7 shows a cross-section elevational view of the device of the invention, in which a row of puncturing units, the units of the suction system and an elevational view of a possible distribution of the electric contacts for said puncturing and suction units are seen.

### Preferred Embodiment of the Invention

As can be seen in the drawings, the device for continuous measuring of an analyte of the present invention is structured, according to a preferred embodiment of the invention, from a fixed element (1) which is placed on the user's skin by an adhesive or mechanical means of the elastic band or watch-strap type or others and comprising an intelligent core or processor and a disposable exchangeable element (2) which is coupled to the fixed element (1) and which incorporates the system performing the sampling or automated mechanical multi-puncturing system comprising a plurality of puncturing units.

Said puncturing units, susceptible of being programmed for the activation thereof in a determined time instant in turn comprise the micro-needles (3), means for driving said micro-needles (3) allowing them to reach different depths, the biosensors with the samples of reagent for mixing with the analyte and the electrodes (13) for the reading and the electric contacts (20) necessary for the operation of said driving means.

Specifically, and according to a possible practical embodiment of the invention shown in the drawings, the fixed element (1) has a central area of preferably rigid or semi-rigid insulating material to facilitate attaching the disposable element (2) and an exterior of semi-elastic insulating material to allow a correct placement on and adhesion to the skin, the electronic part for controlling the disposable element (2) being in the central area, which central part comprises a processing and control unit of the mechanisms for activating and deactivating the driving means and the suction system (19), a data processing unit which allows capturing analog readings coming from an interstitial or blood glucose measurement system, an intelligent core having a base for gathering, translating, interpreting and transmitting data (RDTIT) to the exterior device for reading and active two-directional interaction (DELIBA).

Thus, the central area has a hole in its center to house the disposable element (2) which will be fixed by means of clipping, gripping or another suitable method and will have a preferably rectangular shape, there being according to a possible preferred embodiment in its longer sides the contacts of connection to the disposable element (2) and in the shorter sides the clipping or gripping system for the attachment between the fixed element (1) and the disposable element (2).

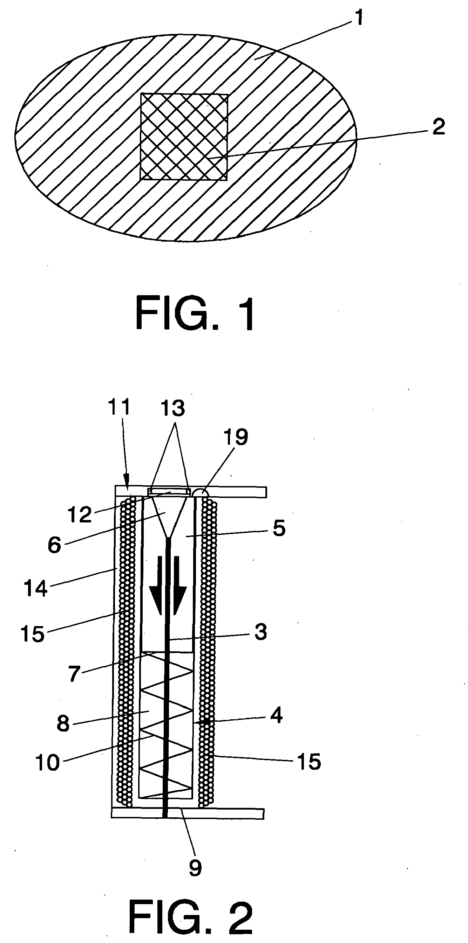

As can be seen in the drawings, especially in the Figures 1 and 2, the disposable element (2) is mechanically coupled to the fixed element (1), being electrically connected to said fixed element (1) through the electric contacts (20) located at the sides thereof.

As has been said, the disposable element (2) incorporates the automated mechanical multi-puncturing system comprising the puncturing units, which are in turn structured from three compartments:
- a cylindrical central compartment (4) comprising:
   o a micro-needle (3) surrounded in its upper portion by a piece made of ferromagnetic material (5) which in its upper pole ends in an conical-shaped empty space (6) intended to be filled with the fluid gathered in the sample and the lower pole of which has a flat cylindrical base (7) serving as a stop as the micro-needle (3) advances.
   o in the lower portion of the central compartment (4) there is a space (8) demarcated by the base (7) and the lower base (9) of said central compartment (4) and which will be passed through by the micro-needle (3) together with the piece made of ferromagnetic material (5) in the advancement thereof towards the sampling, an advancement which will be proportional to the intensity of the induced field such that the depth at which said micro-needle (3) must be introduced according to each case can be controlled. On the other hand, there is in said space (8) an elastic spring (10) such as a spring or the like which allows recovering the micro-needle (3) bringing it to its resting state in the upper portion of the central compartment (4).
   o Likewise, to enable puncturing, the lower base of the central compartment (4) comprises a small opening (not depicted) for the passage of the micro-needle (3) in its displacement for puncturing and obtaining the analyte of blood or interstitial fluid sample.
- an upper compartment (11) located above the central compartment (4) and more specifically on the empty space (6) with the sample analyte and which according to a preferred embodiment of the invention in turn comprises:
   o a biosensor formed by:
      ▪ a separation membrane between the central compartment (4) and the upper compartment (11) which filters elements with molecular weight higher than glucose;
      ▪ an immobilized enzymatic reactive component (12) oxidizing glucose releasing hydrogen peroxide;
      ▪ a second membrane allowing the passage of the hydrogen peroxide which is oxidized in electrodes (13) which in turn gather the potential difference generated, proportional to the amount of glucose.
- an outer compartment (14) enveloping the central compartment (4) by means of a preferably cylindrical space and containing the means for driving the micro-needle (3), which in this case consist of a winding (15) for inducing an electromagnetic field on the piece made of ferromagnetic material (5) integral to said micro-needle (3) such that the latter is displaced along the entire length of said central compartment (4) to the sampling position.

According to a possible embodiment of the invention and in order to save energy, the puncturing units comprise at least one magnet (not depicted) located bottom wise, for example, in the lower area of the central compartment (4) and/or the outer compartment (14) which would allow maintaining the micro-needle (3) in its lowest position as a result of the magnetic attraction thereof while the sampling is performed without the need of inducing a magnetic field in the winding (15), it therefore involves energy saving.

Likewise, the puncturing unit is susceptible of having, together with the above, a second magnet (not depicted) located top wise serving to maintain the micro-needle (3) in resting position, i.e., in an upper position, at the ready for the induction of magnetic field in the winding (15) which commands the puncturing.

As the result of using these magnets, it can even do without an elastic spring (10) which allows recovering the micro-needle (3) or, according to another possible embodiment, coexists with the latter for the purpose of reinforcing the function thereof, the objective of said magnets always being to reduce the amount of magnetic flow used, i.e., the amount of magnetic field induced which will lead to energy saving and therefore to a longer duration of the electric power supply system.

In both cases said magnets could have, for example, a toroidal shape and be housed inside the central compartment (4) and/or the outer compartment (14), although their location and dimensions will generally be those which allow them to carry out the function for which they are designed.

On the other hand, in a manner independent from the puncturing units there is the suction system supplied by means of windings or electromagnetic induction systems, and which is responsible for aiding the sample extraction when this is performed at interstitial level or when it is required, which will be programmable from the control system. More specifically, said suction system comprises on one hand suction pistons (21) outside said puncturing units but connected to them through suction channels (19), which will be responsible for sucking of the sample itself.

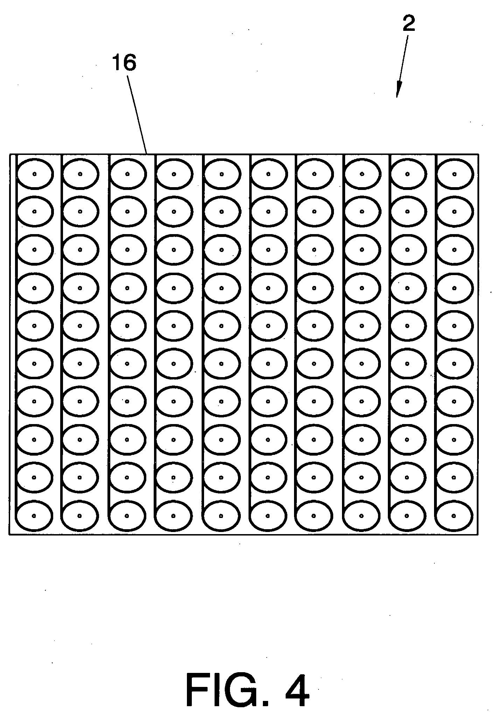

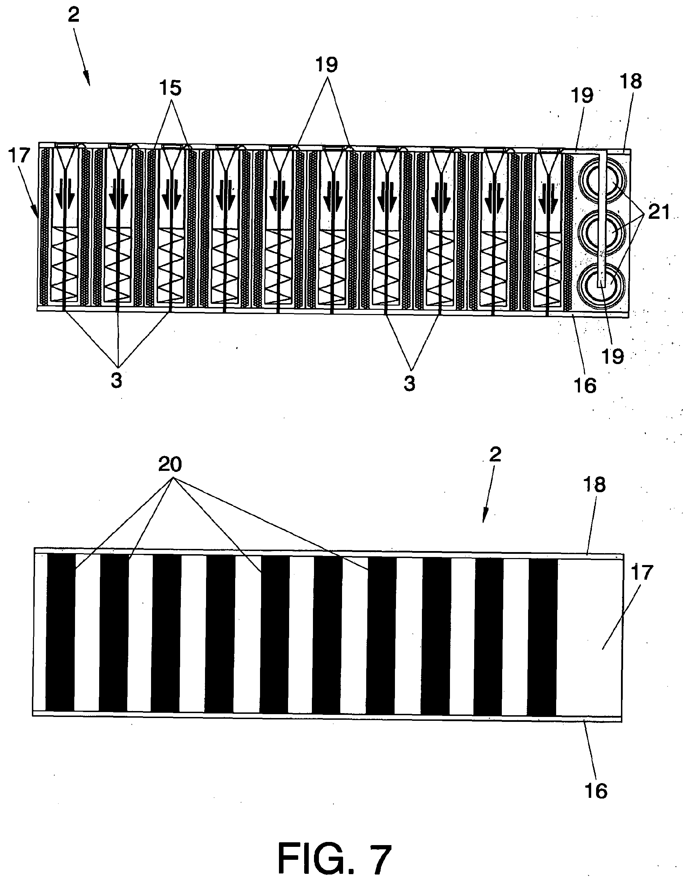

According to the above, a possible practical embodiment of the disposable element (2) of the invention wherein the puncturing elements are arranged in a matrix of 10x10, it could comprise the following elements:
o 100 micro-needles (3)
o 100 windings (15) or electromagnetic induction systems for driving it, and with automatic return;
o 100 enzymatic reactive samples (12) for mixing with interstitial fluid.
o 100 pairs of electrodes (13) for the reading of the potential difference of the oxidative reaction.
o A suction system comprising 3 pistons operated by means of windings or electromagnetic induction systems located as a device coupled to the puncturing units in a physically and functionally independent manner;
Wherein each side or edge of the disposable element (2) has 10 electric contacts (20) (Figure 7):
▪ 10 positive poles for the operation of the windings (15), 1 per row.
▪ 10 negative poles for the operation of the windings (15), 1 per column.
▪ 10 positive poles for the glucose sensors, 1 per row,
▪ 10 negative poles for the glucose sensors, 1 per column.
▪ from 2 to 6 poles for exciting the suction system (19). Therefore, from the constructive point of view and according to a possible practical embodiment of the invention, the disposable device (2) comprises the following three parts:
   a) a lower cover (16) like the one shown in Figure 4 having dimensions of (20 x 20 x 0. 2 mm) which houses:
      - 10 negative contacts to supply power to the windings (15); and
      - 100 openings to allow the micro-needles (3) to penetrate the skin.

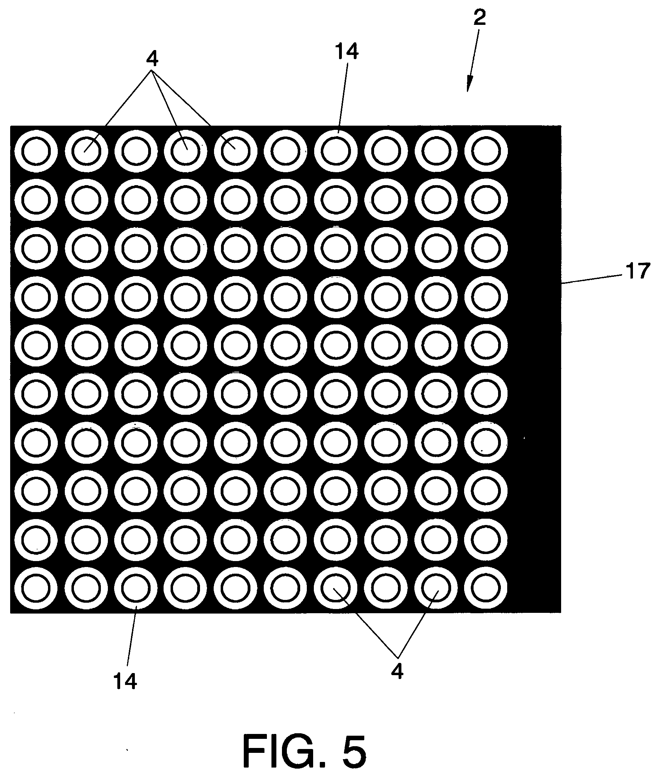

      In another not depicted embodiment, a thin membrane of a shape memorizing material could be placed between the lower layer and the lower base of the central compartment (4) of the disposable element (2) which is passed through by the micro-needles (3) in its actuation. This would have, on one hand, the function of sealing the communications of the micro-needle with the outside and maintaining the sterilization of the same until its activation and on the other hand allows isolating the space (8) of the central compartment (4) after the micro-needle returns to its resting position, once aseptic contamination is made with the biological means and the analyte is extracted.
   b) A central part (17) like the one shown in Figure 5, (20 x 20 x 6 mm) which houses:
      - 100 micro-needles (3); and
      - 100 windings (15) or electromagnetic induction systems for driving them with their corresponding elastic springs (10) for their automatic return.
      - 3 windings or electromagnetic induction systems to supply power to the sample suction system (19).

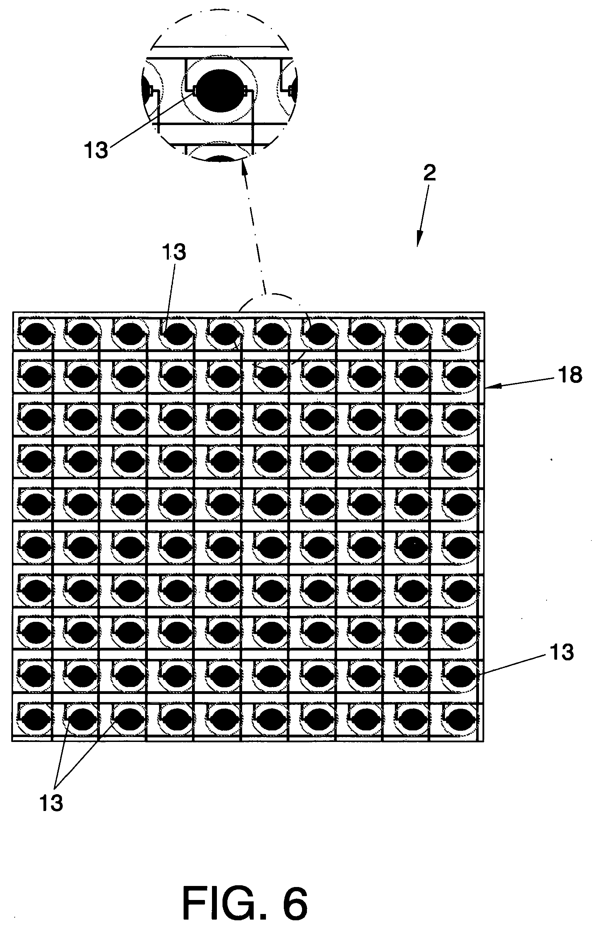

      This central layer which forms the main element will be made, according to a preferred embodiment, of a non-ferromagnetic material such as for example aluminium or epoxy resin.
      Likewise, it will have openings for placing the windings (15) of actuation. Said windings (15) will not be at the same level with the block, but they will protrude for a few hundredths of a millimeter so that once upper and lower covers are placed, they exert certain pressure on the latter to assure the correct electric power supply contact which will be located in said covers.
   c) an upper cover (18) like the one shown in Figure 6, (20 x 20 x 0.2 mm) which houses:
      - 100 sensors formed by as many samples of enzymatic reactive components (12) with their corresponding electrodes (13).
      - 10 positive contacts for supplying power to the windings (15) of the central part.
      - 10 negative contacts for sensors.
      - 10 positive contacts for sensors; and
      - The circuitry interconnections for its communications with the electronic module.

Therefore, the upper cover (18) similar to the lower cover (16) in terms of the constructive material will contain the biosensors comprising the substances which, together and mixed with the blood or interstitial fluid, will facilitate a characteristic measurement current to the incorporated sensors, made of specific materials, also in said cover, for the measurement by the processor of the fixed electronic part. The upper cover (18) will also be formed by more than one superimposed surface, multilayer electronic board, for the purpose of allowing the crossing of electrical connections at different levels, to contain all the buses necessary.

On the other hand, the electric interconnections which will reach the vertexes of the upper and lower covers (16, 18) must be elongated to the profile of the respective edges so that the electric contacts (20) are at the 4 sides of the disposable device, with the purpose of facilitating the electric contact with the fixed part of the apparatus.

Furthermore, said upper and lower covers (16, 18) will have the ducts suitable for transmitting pressure by the 3 suction devices of the suction system (19).

Therefore, in a normal operation of the device of the invention, the control means will send the appropriate activation signals of one or several of the puncturing units with the desired and/or established frequency, puncturing units will obtain the sample either at interstitial level or vessel level as required, said means controlling the induced current which reaches the micro-needles (13) and therefore the depth at which the puncture will be performed, as well as the activation or non-activation of the suction system (19).

Thus, as has been seen in the preferred embodiment described and shown in the drawings, the disposable element (2) will have several dimensions in plan views of (20 x 20 mm), and in elevational views of 6.4 mm in height, 40 electric contacts 10 distributed at each side of the square being needed, although another arrangement could also be possible, such as for example that which arrange 20 contacts in the opposite faces, and the other free faces can be used for anchoring to the fixed element (1).

On the other hand, in terms of the electronics of the fixed element (1) and according to a possible embodiment of the invention in accordance with the aforementioned preferred embodiment for the disposable element (2), the same has to comprise the following non depicted parts:
a. a processing unit for the control and operation of the mechanical part of the device.
b. a data processing unit which allows capturing analog readings coming from an interstitial or blood glucose measurement system.
c. an intelligent core for gathering, translating, interpreting and transmitting the data RDTIT to the exterior device for reading and active two-directional interaction (DELIBA).
d. an integrated circuit for wireless communication such as a BlueTooth® system or the like.
e. a multiplexer system for powering the means for driving the micro-needles (3).
f. multiplexer system for selecting the different sensors and routing towards the processor.
g. one or two 3 V lithium batteries.

For the aforementioned possible practical embodiment in which the micro-needles (3) are arranged in a matrix of 10 x 10 this electronics will at least be made up of a 4 multiplexer system supplied and controlled by an address bus of at least 4 bits from a processor, two of which will allow a routing towards a determined pair of metal sensors (x, y) of the matrix of the disposable element (2) for reading the data. Likewise, it will also serve to direct the power supply to a determined winding (15) responsible for the actuation of the corresponding micro-needle inside the matrix, not ruling out the need of using any other element for supplying additional power to said windings.

It is therefore deduce that the address bus of the (x) multiplexer of the sensors will have the same information as that of the (x) windings (15), and thus with the (y) multiplexers, being able to use the same address bus. Therefore, if 4 bits are needed for the (x) and 4 more for the (y), using a bus of 1 byte (8 bits) of the processor for addressing the matrix of the disposable will be sufficient.

The processor will maintain the addressing status of the byte throughout the process from the start to finish, being able to use an extra "hold" bit where appropriate. Said extra bit known as "hold" is used so that the multiplexers maintain their addressing while said bit is activated, allowing the processor to change the status of the address bus for other operations without affecting the addressing status of the multiplexers of the matrix.

Said processor will further direct and exchange data with a memory having a sufficient capacity for storing data of the samples, with the related metadata thereof such as the day and time, coordinates of the micro-needle (3), batch, user, license, and those considered appropriate.

Said memory will be a flash type memory, in a preferred embodiment given its capacity for storing data after it is disconnected from the power supply, other types are not ruled out if they are considered as more appropriate.

On the other hand, and if the measurements allow it, instead of it being welded to the board said electronics will have a connector for extracting the memory. And if the same is desired for the processor, a base which allows the extraction thereof for its possible reprogramming and adjustment by the technical staff. Instead of the fastening socket for the processor, a system of connectors which allows connecting the fixed device, for example through mini USB, with a computer for said tasks is not ruled out.

Said fixed electronic part will contain, as has been said, a device for connecting with the outside in a wireless manner, preferably with Bluetooth technology which will be communicated with the processor by means of a data bus. Said Bluetooth® device must allow typical safety characteristics, such as the terminal presentation and the linking between them.

Finally, the power supply system of the device for continuous measurement of the invention could be formed according to a possible embodiment by 3V coin cell batteries, not ruling out other energy generation systems such as for example, peltier cells in reverse which convert the body heat into a potential difference capable of being stored in condensers or rechargeable batteries of the fixed electronic part.

Another advantage of the present invention is its versatility when being used for obtaining and analyzing other type of samples given its capacity to select the moment over time and/or frequency of the analysis and to control parameters such as the depth of the puncture and the possibility of extracting the sample by means of suction if necessary. Thus, it will be sufficient to intercalate specific puncturing units to perform other type of measurements, such as for example that of fructosamine for the purpose of combining measurements from different indicators and thus obtaining a more complete and efficient tracking of the diabetes.

## Claims

1. Device for continuous measuring of an analyte comprising a fixed element (1) and a disposable and exchangeable element (2), wherein the fixed element (1) Is placed on the user's skin and which in turn comprises an intelligent core for controlling the disposable and exchangeable element (2) coupled thereto, wherein said disposable element (2) incorporate an automated multi-puncturing system programmable over time comprising a plurality of puncturing units for sampling, wherein said puncturing units comprises a micro-needle (3) and means for driving said micro-needle (3) comprising a winding (15) responsible for inducing an electromagnetic field on a ferromagnetic material (5) integral to the micro-needle (3) such that it is displaced to the sampling position In a manner proportional to the intensity of the field induced such that puncturing at different depths Is allowed **characterised in that** the puncturing units are formed by:
- A central compartment (4) comprising:
o the micro-needle (3) surrounded in its upper portion by the piece made of ferromagnetic material (5) In the upper pole of which there is an empty space (6) intended to be filled with the sample fluid and the lower pole of which has a base (7) serving as a stop as the micro-needle (3) advances through a space (8) of the lower portion of the central compartment (4),
o an opening in the lower base (9) for the passage of the micro-needle (3) in its displacement; and
o an elastic spring (10) which allows recovering the micro needle (3) bringing it to its resting state In the upper portion of the central compartment (4), and/or a magnet located top wise serving to maintain the micro-needle (3) in the upper position at the ready for the induction of magnetic field in the winding (15) which command the puncturing.
and
- an upper compartment (11) located above the central compartment (4) comprising a biosensor;

2. Device for continuous measuring of an analyte according to claim 1. **characterized in that** the biosensor comprises:
▪ a separation membrane between the central compartment (4) and the upper compartment (11) for filtering elements with molecular weight higher than glucose;
▪ an immobilized enzymatic reactive: component (12) oxidizing glucose releasing hydrogen peroxide;
▪ a second membrane allowing the passage of the hydrogen peroxide which is oxidized in electrodes (13) which in turn gather the potential difference generated, proportional to the amount of glucose.

3. Device for continuous measuring of an analyte according to claim 1, **characterized in that** it comprises a suction system comprising suction pistons (21) separated from the puncturing units but connected to them through suction channels (19).

4. Device for continuous measuring of an analyte according to the preceding claims, **characterized in that** it comprises:
a) a processing unit for controlling and operating the means for driving the micro-needles (3) and the suction system (19);
b) a data processing unit for capturing the analog readings coming from the interstitial or blood glucose measurement;
c) an intelligent core for gathering, translating, interpreting and transmitting the data to an exterior device for reading and active two-directional interaction;
d) an integrated circuit for wireless! communication with the exterior device;
e) a multiplexer system for storing the power of the means for driving the micro-needles (3);
f) multiplexer system for selecting the different sensors and routing towards the processor, and
g) an electric supply system. ,

5. Device for continuous measuring of an analyte according to the preceding claims, **characterized in that** the puncturing units disposable element (2) are arranged in a matrix of 10x10 which comprises:
o 100 micro-needles (3) with as many windings (15) or electromagnetic induction systems for driving it, and returning means;
o 100 biosensors:
o A suction system comprising 3 suction pistons (21) operated by means of windings or electromagnetic induction systems;
o At least 4 multiplexers supplied and controlled by an address bus of at least 4 bits from the processor.

6. Device for continuous measuring of an analyte according to claim 1, **characterized in that** it comprises a membrane of a shape memorizing material in the lower base of the central compartment (4) of the disposable element (2) which is passed through by the micro-needles (3) In its actuation.

7. Device for continuous measuring of an analyte according to claim 1, **characterized in that** the disposable element (2) Is fixed to the fixed element (1) by means of clipping or gripping.

8. Device for continuous measuring of an analyte according to claim 1, **characterized in that** the disposable element (2) has a rectangular shape, having at its longer sides electric contacts (20) of connection to the disposable element (2) and in the shorter side the clipping or gripping system for attaching to the fixed element (1).

9. Device for continuous measuring of an analyte according to claim 1, **characterized in that** the fixed element (1) has a central area of rigid or semi-rigid insulating material to facilitate attaching the disposable element (2) and an exterior of semi-elastic insulating material to allow a correct placement on and adhesion to the skin, the electronic part for controlling the disposable element (2) being In the central area.

10. Device for continuous measuring of an analyte according to claim 1, **characterized in that** the fixed element (1) Is placed on the user's skin by means of an adhesive means.

11. Device for continuous measuring of an analyte according to claim 1, **characterized in that** the fixed element (1) is placed on the user's skin by means of an elastic band or strap.

12. Device for continuous measuring of an analyte according to claim 1, **characterized in that** the puncturing units comprise a magnet located bottom wise to maintain the micro-needle (3) In its lowest position as a result of the magnetic attraction thereof while sampling is performed.

13. Device for continuous measuring of an analyte according to claims 1 and / or 12. **characterized in that** the magnets are toroidal shaped.

## Patentansprüche

1. Vorrichtung zur kontinuierlichen Messung eines Analyten, umfassend ein feststehendes Element (1) und ein Einweg- und austauschbares Element (2), wobei das feststehende Element (1) auf der Haut des Benutzers platziert wird und welches wiederum einen intelligenten Kern zur Kontrolle des wegwerfbaren und austauschbaren Elements (2), das daran gekoppelt ist, umfasst, wobei das Einweg-Element (2) ein automatisiertes, über die Zeit programmierbares Multi-Durchstoß-System inkorporiert, welches eine Vielzahl von Durchstoßeinheiten zur Probenentnahme umfasst, wobei die Durchstoßeinheiten eine Mikronadel (3) und Mittel, um die Mikronadel (3) zu treiben, umfassend eine Wicklung (15), die für die Induktion eines elektromagnetischen Feldes auf ein ferromagnetisches Material (5) integral zu der Mikronadel (3) verantwortlich ist, so dass sie auf eine zur Intensität des induzierten Feldes proportionale Weise zu der Probenentnahmeposition verlagert wird, so dass Durchstoßen auf verschiedene Tiefen ermöglicht wird, umfasst, dadurch charakterisiert, dass die Durchstoßeinheiten gebildet sind durch:
- Eine zentrale Kammer (4), umfassend:
- die Mikronadel (3), deren oberer Teil von dem aus ferromagnetischen Material (5) gemachten Stück umgeben ist, in dessen oberem Pol sich ein leerer Raum (6) befindet, der mit der Probenflüssigkeit gefüllt werden soll, und dessen unterer Pol eine Basis (7) hat, die als ein Stopp dient, wenn die Mikronadel (3) durch einen Raum (8) im unteren Teil der zentralen Kammer (4) vorrückt,
- eine Öffnung in der unteren Basis (9) für die Passage der Mikronadel (3) in deren Verlagerung; und
- eine elastische Feder (10), die das Zurückholen der Mikronadel (3) erlaubt und sie in ihren Ruhezustand im oberen Teil der zentralen Kammer (4) bringt, und/oder einen Magneten, der oberhalb angeordnet ist und dazu dient, die Mikronadel (3) in der oberen Position für die Induktion des magnetischen Felds in der Wicklung (15), die das Durchstoßen befiehlt, bereit zu halten.
und
- eine obere Kammer (11), die über der zentralen Kammer (4) angeordnet ist, umfassend einen Biosensor.

2. Vorrichtung zur kontinuierlichen Messung eines Analyten nach Anspruch 1, **dadurch gekennzeichnet, dass** der Biosensor umfasst:
- eine Trennmembran zwischen der zentralen Kammer (4) und der oberen Kammer (11) zur Filtrierung von Elementen mit einem höheren Molekulargewicht als Glukose;
- eine immobilisierte enzymatische reaktive Komponente (12), die durch die Oxidation von Glukose Wasserstoffperoxid freisetzt;
- eine zweite Membran, die die Passage des Wasserstoffperoxids, welches in Elektroden (13) oxidiert wird, die wiederum die generierte Potentialdifferenz sammeln, proportional zur Menge der Glukose ermöglicht.

3. Vorrichtung zur kontinuierlichen Messung eines Analyten nach Anspruch 1, **dadurch gekennzeichnet, dass** sie ein Absaugsystem umfasst, welches Absaugkolben (21), die von den Durchstoßeinheiten getrennt, aber durch Absaugkanäle (19) mit ihnen verbunden sind, umfasst.

4. Vorrichtung zur kontinuierlichen Messung eines Analyten nach den vorherigen Ansprüchen, **dadurch gekennzeichnet, dass** sie umfasst:
a) eine Prozessoreinheit zur Kontrolle und Betreibung der Mittel zum Treiben der Mikronadeln (3) und des Absaugsystems (19);
b) eine Datenprozessoreinheit zur Erfassung der analogen Messwerte aus der interstitiellen oder Blutglukosemessung;
c) einen intelligenten Kern zum Sammeln, Übersetzen, Interpretieren und Übermitteln der Daten an eine externe Vorrichtung zum Auslesen und zum aktiven Austausch in beide Richtungen;
d) eine integrierte Schaltung zur drahtlosen Kommunikation mit der externen Vorrichtung;
e) ein Multiplexer-System zur Speicherung der Leistung der Mittel zum Treiben der Mikronadeln (3);
f) Multiplexer-System zur Auswahl der verschiedenen Sensoren und zur Leitung an den Prozessor; und
g) eine elektrische Versorgungsanlage.

5. Vorrichtung zur kontinuierlichen Messung eines Analyten nach den vorherigen Ansprüchen, **dadurch gekennzeichnet, dass** die Durchstoßeinheiten des Einweg-Elements (2) in einer 10x10 Matrix angeordnet sind, die umfasst:
- 100 Mikronadeln (3) mit ebenso vielen Wicklungen (15) oder elektromagnetischen Induktionssystemen um sie anzutreiben, und Rückholmittel;
- 100 Biosensoren;
- ein Absaugsystem umfassend 3 Absaugkolben (21), die durch Wicklungsmittel oder elektromagnetische Induktionssysteme betrieben werden;
- mindestens 4 Multiplexer, die von einem Adressbus von mindestens 4 Bits des Prozessors bereitgestellt und kontrolliert werden.

6. Vorrichtung zur kontinuierlichen Messung eines Analyten nach Anspruch 1, **dadurch gekennzeichnet, dass** sie eine Membran aus formmemorierendem Material in der unteren Basis der zentralen Kammer (4) des Einweg-Elements (2) umfasst, die von den Mikronadeln (3) bei ihrer Betätigung durchdrungen wird.

7. Vorrichtung zur kontinuierlichen Messung eines Analyten nach Anspruch 1, **dadurch gekennzeichnet, dass** das Einweg-Element (2) an das feststehende Element (1) durch klammernde oder greifende Mittel fixiert ist.

8. Vorrichtung zur kontinuierlichen Messung eines Analyten nach Anspruch 1, **dadurch gekennzeichnet, dass** das Einweg-Element (2) eine rechteckige Form hat, wobei es an seinen längeren Seiten elektrische Kontakte (20) zur Verbindung mit dem Einweg-Element (2), und an der kürzeren Seite das Klammer- oder Greifsystem zum Anbringen an das feststehende Element (1) hat.

9. Vorrichtung zur kontinuierlichen Messung eines Analyten nach Anspruch 1, **dadurch gekennzeichnet, dass** das feststehende Element (1) einen zentralen Bereich aus rigidem oder halb-rigidem Isoliermaterial zur Erleichterung des Anbringens des Einweg-Elements (2) und ein Äußeres aus halb-elastischem Isoliermaterial hat, um eine korrekte Platzierung auf und Adhäsion an die Haut zu erlauben, wobei der elektronische Teil zur Kontrolle des Einweg-Elements (2) in dem zentralen Bereich ist.

10. Vorrichtung zur kontinuierlichen Messung eines Analyten nach Anspruch 1,
**dadurch gekennzeichnet, dass** das feststehende Element (1) mittels eines haftenden Mittels auf der Haut des Benutzers platziert wird.

11. Vorrichtung zur kontinuierlichen Messung eines Analyten nach Anspruch 1,
**dadurch gekennzeichnet, dass** das feststehenden Element (1) mittels eines elastischen Bands oder Riemens auf der Haut des Benutzers platziert wird.

12. Vorrichtung zur kontinuierlichen Messung eines Analyten nach Anspruch 1,
**dadurch gekennzeichnet, dass** die Durchstoßeinheiten einen Magneten, der unterhalb angebracht ist, umfassen, um die Mikronadel (3) während die Probenentnahme durchgeführt wird als Folge dessen magnetischer Anziehung in ihrer untersten Position zu halten.

13. Vorrichtung zur kontinuierlichen Messung eines Analyten nach Anspruch 1 und/oder 12, **dadurch gekennzeichnet, dass** die Magneten ringförmig sind.

## Revendications

1. Dispositif pour la mesure en continu d'un analyte comprenant un élément fixe (1) et un élément jetable et échangeable (2), dans lequel l'élément fixe (1) est placé sur la peau de l'utilisateur et comprend un coeur intelligent pour la commande de l'élément jetable et échangeable (2) auquel il est couplé, dans lequel ledit élément jetable (2) comprend un système de multi-perforation automatique qui est programmable dans le temps et qui comprend une pluralité d'unités de perforation pour la collecte d'échantillons, lesquelles unités de perforation comprennent une micro-aiguille (3) et un moyen d'entraînement de ladite micro-aiguille (3) comprenant un bobinage (15) destiné à induire un champ électromagnétique dans un matériau ferromagnétique (5) solidaire de la micro-aiguille (3) de sorte à déplacer celle-ci en position d'échantillonnage de manière proportionnelle à l'intensité du champ induit pour permettre la perforation à différentes profondeurs, **caractérisé en ce que** les unités de perforation sont formées par :
- un compartiment central (4) comprenant :
o la micro-aiguille (3) entourée dans sa partie supérieure par la pièce en matériau ferromagnétique (5), dans le pôle supérieur de laquelle est prévu un espace vide (6) destiné à être rempli par un échantillon de fluide et dont le pôle inférieur est pourvu d'une base (7) servant de butée lorsque la micro-aiguille (3) avance à travers un espace (8) de la partie inférieure du compartiment central (4),
o une ouverture dans la base inférieure (9) pour le passage de la micro-aiguille (3) dans son déplacement ; et
o un ressort élastique (10) qui permet de récupérer la micro-aiguille (3), la ramenant dans son état de repos dans la partie supérieure du compartiment central (4), et / ou un aimant positionné de manière haute servant à maintenir la micro-aiguille (3) dans la position supérieure, prête pour l'induction d'un champ magnétique dans le bobinage (15) qui commande la perforation ;
et
- un compartiment supérieur (11) positionné au-dessus du compartiment central (4) comprenant un biocapteur.

2. Dispositif pour la mesure en continu d'un analyte selon la revendication 1, **caractérisé en ce que** le biocapteur comprend :
- une membrane de séparation entre le compartiment central (4) et le compartiment supérieur (11) pour filtrer des éléments d'un poids moléculaire supérieur à celui du glucose ;
- un composant immobilisé réactif aux enzymes (12) qui oxyde le glucose en libérant du peroxyde d'hydrogène ;
- une deuxième membrane autorisant le passage du peroxyde d'hydrogène, lequel est oxydé dans des électrodes (13) qui collectent alors la différence de potentiel générée, proportionnelle à la quantité de glucose.

3. Dispositif pour la mesure en continu d'un analyte selon la revendication 1, **caractérisé en ce qu'**il comprend un système d'aspiration comprenant des pistons d'aspiration (21) séparés des unités de perforation mais connectés à ces dernières par des canaux d'aspiration (19).

4. Dispositif pour la mesure en continu d'un analyte selon les revendications précédentes, **caractérisé en ce qu'**il comprend :
a) une unité de traitement pour commander et faire fonctionner le moyen d'entraînement des micro-aiguilles (3) et le système d'aspiration (19) ;
b) une unité de traitement des données pour capturer les valeurs analogiques des mesures de glucose interstitiel ou sanguin ;
c) un coeur intelligent pour collecter, traduire, interpréter et transmettre les données à un dispositif externe pour la lecture et l'interaction active bidirectionnelle ;
d) un circuit intégré pour la communication sans-fil avec le dispositif externe ;
e) un multiplexeur pour stocker l'énergie du moyen d'entraînement des micro-aiguilles (3) ;
f) un multiplexeur pour sélectionner les différents capteurs et pour acheminer vers le processeur ; et
g) un système d'alimentation électrique.

5. Dispositif pour la mesure en continu d'un analyte selon les revendications précédentes, **caractérisé en ce que** les unités de perforation de l'élément jetable (2) sont agencées selon une matrice 10x10 qui comprend :
- 100 microaiguilles (3) avec autant de bobinages ou de systèmes d'induction électromagnétique pour l'entraîner, et de moyens de rappel :
- 100 biocapteurs ;
- un système d'aspiration comprenant 3 pistons d'aspiration (21) manoeuvrés au moyen de bobinages ou de systèmes d'induction électromagnétique ;
- au moins 4 multiplexeurs fournis et commandés par un bus d'adresses d'au moins 4 bits du processeur.

6. Dispositif pour la mesure en continu d'un analyte selon la revendication 1, **caractérisé en ce qu'**il comprend une membrane en un matériau à mémoire de forme dans la base inférieure du compartiment central (4) de l'élément jetable (2) qui est traversée par les micro-aiguilles (3) dans son actionnement.

7. Dispositif pour la mesure en continu d'un analyte selon la revendication 1, **caractérisé en ce que** l'élément jetable (2) est fixé à l'élément fixe (1) par encliquetage ou préhension.

8. Dispositif pour la mesure en continu d'un analyte selon la revendication 1, **caractérisé en ce que** l'élément jetable (2) a une forme rectangulaire et a, sur ses côtés les plus longs, des contacts électriques (20) de connexion à l'élément jetable (2) et, dans son côté le plus court, le système d'encliquetage ou de préhension pour la fixation à l'élément fixe (1).

9. Dispositif pour la mesure en continu d'un analyte selon la revendication 1, **caractérisé en ce que** l'élément fixe (1) a une zone centrale en un matériau isolant rigide ou semi-rigide pour faciliter la fixation de l'élément jetable (12) et un extérieur en un matériau isolant semi-élastique pour permettre un placement correct et l'adhésion sur la peau, la partie électronique pour la commande de l'élément jetable (2) étant dans la zone centrale.

10. Dispositif pour la mesure en continu d'un analyte selon la revendication 1, **caractérisé en ce que** l'élément fixe (1) est placé sur la peau de l'utilisateur au moyen d'un adhésif.

11. Dispositif pour la mesure en continu d'un analyte selon la revendication 1, **caractérisé en ce que** l'élément fixe (1) est placé sur la peau de l'utilisateur au moyen d'une bande ou d'une sangle élastique.

12. Dispositif pour la mesure en continu d'un analyte selon la revendication 1, **caractérisé en ce que** les unités de perforation comprennent un aimant positionné de manière basse pour maintenir la micro-aiguille (3) dans sa plus basse position en raison de l'attraction magnétique de celui-ci lorsque la collecte d'échantillon est réalisée.

13. Dispositif pour la mesure en continu d'un analyte selon les revendications 1 et / ou 12, **caractérisé en ce que** les aimants sont de forme toroïdale.
